# EUROPEAN PATENT SPECIFICATION

(11) **EP 2 073 712 B1**
(45) Date of publication and mention of the grant of the patent: **26.05.2010**
(21) Application number: 07842118.7
(22) Date of filing: 10.09.2007
(51) Int. Cl.: A61B 8/00, A61B 8/06

(54) **METHOD AND APPARATUS FOR HANDS-FREE ULTRASOUND**
VERFAHREN UND VORRICHTUNG FÜR FREIHÄNDIGEN ULTRASCHALL
PROCEDE ET APPAREIL D'APPLICATION D'ULTRASONS MAINS LIBRES

(30) Priority: 29.09.2006 US 827476 P
(43) Date of publication of application: 01.07.2009
(73) Proprietor: Koninklijke Philips Electronics N.V., 5621 BA Eindhoven (NL)
(72) Inventor: ERKAMP, Ramon, Q., Purdys, New York 10578 (US); COHEN-SOLAL, Eric, V., Ossiningq, New York 10562 (US); RAJU, Balasundara, I., Tarrytown, New York 10591 (US); AZEVEDO, Jose, M. I., Mahopac, New York 10541 (US)
(74) Representative: Schouten, Marcus Maria
(86) International application number: PCT/US2007/077979
(87) International publication number: WO 2008/042559

(56) References cited:
- EP-A- 0 947 163
- WO-A-02/094373
- WO-A-02/098271
- WO-A-2007/057826
- US-A- 5 394 877
- US-A- 5 598 845
- US-A1- 2005 288 572

## Description

The present application relates to diagnostic arts. It finds particular application in relation to ultrasound monitoring and will be described with particular reference thereto. However, it is to be appreciated that the following will also find application in conjunction with ultrasound imaging, testing, treatment, and the like.

Ultrasound systems are valuable diagnostic tools for providing in real time critical information about the patient's condition such as flow of blood, heart beat, tissue movements, and the like. Typically, the ultrasound diagnostic systems use non-invasive technology based, for example, on the Doppler effect. In such systems, high accuracy of measurements is combined with simplicity of diagnostic procedures. Typically, a medical professional performs ultrasound by using a manual probe or transducer by applying a pressure and changing the position of the probe.

Additionally, the Doppler systems can be used to detect the patient's pulse and measure blood flow in emergency situations, during surgery, in ICU, and the like. In such situations, a presence of a medical professional who is dedicated to the ultrasound machine is required in addition to other medical personnel. Similarly, in many therapeutic applications of ultrasound, an operator is needed to hold the transducer at a specific part of the body. This is costly and, as studies have shown, causes undesirable health deteriorations in the medical personnel such as repetitive stress injury due to the need to apply the constant pressure on the transducer. A direct attachment of the transducer to the patient's skin could avoid the extra workload on medical personnel and make ultrasonic monitoring more attractive.

One approach is to attach an ultrasound transducer to the patient's skin with an adhesive bandage or the like. However, such technique does not soundly acoustically couple the ultrasound transducers with the patient's skin. Another approach is to place a gel pad between the transducers and the skin. Yet another approach is to spread a conventional acoustic coupling gel to the skin and apply the ultrasound transducers over it. The acoustic pad is disadvantageous because air pockets are defined between the pad and fissures in the skin which cause excessive acoustic reflection. Acoustic coupling gel is disadvantageous because it is slippery and might permit movement of the transducer relative to the skin. Suchlike medical devices and systems are disclosed in prior art documents US 5 394 877 A, WO 02 / 094 373 A1, EP 0 947 163 A2, US 5 598 845 A, US 2005 / 0 288 572 A1 and WO 03 / 096 894 A1, for instance.

The present application provides new and improved methods and apparatuses which overcome the above-referenced problems and others.

In accordance with one aspect, a skin-mounted device is disclosed which comprises an acoustic module and an attachment assembly. An acoustically coupling layer affixes and acoustically couples the module to the skin and a flexible covering layer surroundingly extends over the module to permit flexing for conforming to the contour of the skin. An adhesive pad extends peripherally around the coupling layer, wherein the covering layer covers an opposite side of the module from the coupling layer and an inner portion of the adhesive pad is sandwiched between the covering and coupling layers.

In accordance with another aspect, a method of acoustic monitoring is disclosed. An acoustic module is affixed and acoustically coupled with a patient's skin with an acoustic coupling layer. Acoustic signals are transmitted and received with the module to monitor a physiological parameter of the patient.

One advantage resides in a hands-free mechanism which affixes an ultrasound sensor to the skin.

Still further advantages of the present invention will be appreciated to those of ordinary skill in the art upon reading and understanding the following detailed description. In a preferred embodiment of the present invention the acoustic module includes a wireless transducer.

The invention may take form in various components and arrangements of components, and in various steps and arrangements of steps. The drawings are only for purposes of illustrating the preferred embodiments and are not to be construed as limiting the invention.
FIGURE 1 is a diagrammatic illustration of an ultrasound system;
FIGURE 2 is a diagrammatic illustration of a bottom view of an ultrasound assembly
FIGURE 3 is a diagrammatic illustration of another embodiment of an ultrasound system; and
FIGURE 4 is a diagrammatic illustration of a monitoring system.

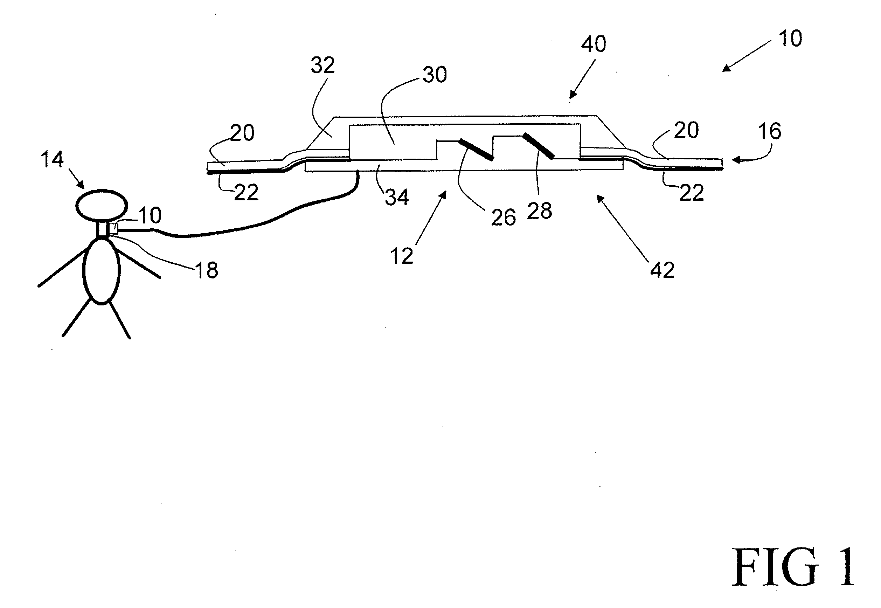

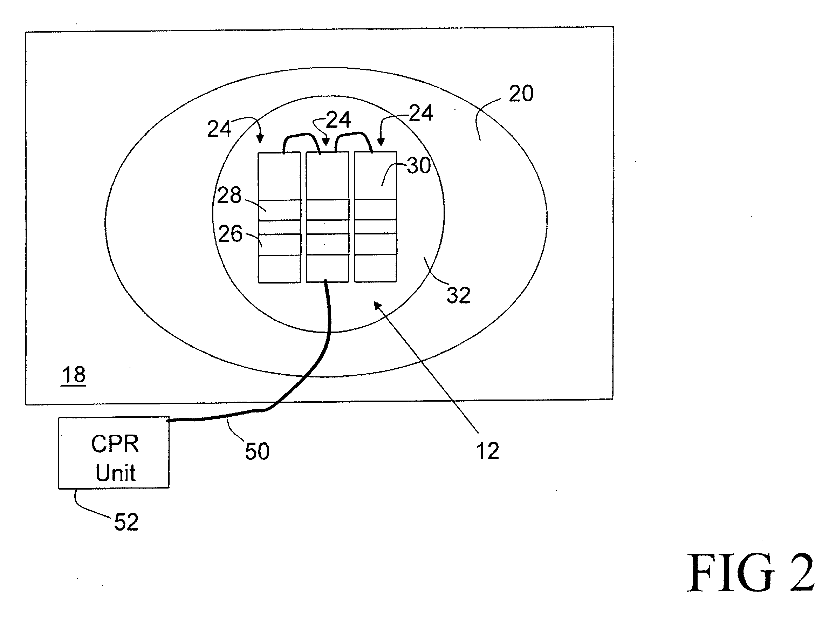

With reference to FIGURES 1 and 2, an ultrasound assembly **10** includes a medical, monitoring or biometric device **12** such as a Doppler effect based ultrasound sensor which, for example, detects, measures, and/or monitors a physiological parameter. The examples of the physiological parameter are flow of blood, heart beat, and tissue movements. The medical device **12** is attached to a patient **14** via an attachment assembly **16** which securely attaches the medical device **12** to a base surface or skin **18** of the patient **14.** More specifically to the first embodiment, the attachment assembly **16** includes a pad **20,** such as a pediatric foam pad, with a layer **22** of adhesive material that adheres the pad **20** with the ultrasound sensor **12** to the skin of the patient. In one embodiment, the ultrasound sensor **12** includes a piezoelectric transducer including an array of piezoelectric modules, elements or crystals **24.** Each piezoelectric module **24** includes a transmitter **26** and a receiver **28** mounted to a former or support **30.** The former **30** holds each module element **24** sufficiently rigid so that the orientation between each pair of transmitter and receiver of each element **24** is fixed. Of course, it is contemplated that the module includes a transceiver. The piezoelectric modules **24** are interconnected with a stiff, but sufficiently flexible covering layer **32** so that the transducers can be contoured to the contour of the patient. Of course, for imaging transducer arrays, all of the transducers need to be kept in a preselected, known relationship to each other. Although a piezoelectric transducer is illustrated in the exemplary embodiment, an ordinary person skilled in the art will appreciate that any other type of component or material that exhibits piezoelectric attributes may be used so that when the material is energized it produces a mechanical effect. Any other ultrasound transducers such as for example CMUT (capacitive micromachined ultrasonic transducer) may be used as well.

The modules **24** are enclosed in the flexible covering layer **32,** which in one embodiment, is a flexible silicone material. For example, the covering layer **32** includes a layer of a rigid adhesive material such a T2® silicone that covers a top or first surface **40** of the ultrasound assembly **10.** The attachment assembly **16** also includes an acoustic coupling layer **34** which is disposed at a lower or second surface **42** of the ultrasound assembly **10** to make direct contact with the skin **18.** For example, the coupling layer **34** includes a layer of an elastomeric material which is sufficiently tacky or sticky so that it adheres to the skin **18** and, which, although sufficiently firm so as to prevent transducer slippage, is sufficiently fluid so that it penetrates and fills fissures in the skin, eliminating air pockets. The material of the coupling layer **34** has an acoustic transmissivity which transmits the ultrasound signals at substantially the speed of sound in human tissue (water), with reasonably low attenuation and an acoustic impedance near that of tissue. An example of a suitable material is Sylgard® which is a dielectric gel manufactured by Dow Corning. Such gel represents a special encapsulant that can cure to an extremely soft material such as skin. The cured gel retains much of the stress, relief and self healing qualities of the liquid while providing the dimensional stability of an elastomer. The gel is formulated and cured such that the surface of the cured gel is naturally tacky which allows the gel to gain physical adhesion without the need for primers.

In one embodiment, the coupling layer **34** is substantially rigid with the strong adhesive qualities. In another embodiment, the sensor is strongly pushed onto the skin with a peripheral adhesive attachment.

With continuing reference to FIGURE 2, conductor **50** connects the ultrasound sensor **12** to a monitor unit, such as a CPR unit **52.** Optionally, the ultrasound assembly **10** can include a battery, an ultrasound, a transmit/receive controller, and a wireless transmitter for wireless communications such as body coupled communications, Bluetooth, or the like. The circuitry for interpreting the ultrasound echoes, such as the circuitry for connecting the echo data into pulse rate, can be mounted in part or in full on the ultrasound assembly **10.** As another option, a wireless communication and battery module can be tethered to the ultrasound assembly.

Directly adhering the ultrasound assembly **10** onto the patient **14** prevents movement of the ultrasound sensor **12** while sensing the Doppler energy in, for example, the carotid artery. The ultrasound assembly **10** is isolated from sensing false Doppler energy generated because of transducer movement. Further, the adhesiveness of the attachment assembly **16** provides the medical personnel with a degree of freedom. Specifically, the medical professional may place the ultrasound assembly **10** on the patient **14** without having to hold it in place while taking the pulse. Future pulse checks can be performed without further personal attention to the transducer assembly. The large adhesive contact area reduces motion due to tension in the sensor cable. The Sylgard® coupling material provides good acoustic contact while eliminating air bubbles at the skin interface, and is also non-perishable so that long term storage is not an issue. The lower sound speed in this material causes beam refraction allowing for a flatter patch design because the crystals do not have to be angled as steep.

The refraction increases the ultrasound beam angle out of the sensor which is needed to get a strong Doppler component. This makes the overall design even more like a flat patch because the piezo elements do not have to be steeply angled to get a steep exit angle of the beam from the sensor.

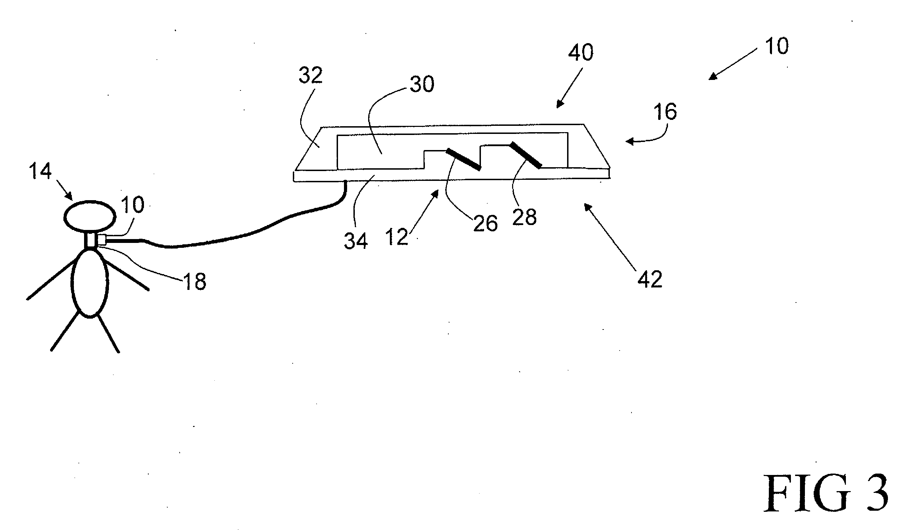

With reference to FIGURE 3, the piezoelectric modules **24** arc disposed between the covering and acoustic coupling layers **32, 34.** The pad **20** with the adhesive layer **22** of FIGURES 1 and 2 is omitted, which is not in accordance with the invention. The acoustic coupling layer **34** includes a sticky elastomeric silicone material such as Sylgard®. The flexible covering layer **32** includes a rigid non-sticky silicone material such as T2®. In another embodiment, the covering and acoustic coupling layers **32, 34** both include elastomeric silicone material such as Sylgard®. The covering layer **32** includes a thin outer layer of non sticky material such as a Teflon layer to facilitate manual handling.

The Sylgard 527® provides good adhesive attachment to the skin, ensures there are no air gaps, can be removed and reapplied, and provides good acoustic coupling even under dry skin conditions. Of course it is contemplated that other coupling materials with appropriate acoustic properties as well as other attachment could be used. As another example, the mixing properties of Sylgard 527® can be modified to balance rigidity/liquidity/tackiness. Generally, the coupling material needs to have low acoustic attenuation of ultrasonic wave and good acoustic contact, for example, a liquid like surface that can fill crevasses and contours of skin. The air pockets at contact surface must be avoided, for example, with a soft sticky material.

The transmitters/receivers **26, 28** are mounted in each module **24** at a predetermined orientation with respect to the patient's skin surface. In the alternative, a single transmitter and multiple receivers or multiple transmitters and a single receiver may be mounted within each module.

Generally, it is desirable to orient the transmitters/receivers **26, 28** in the module **24** so that, for example, the blood flow up the carotid artery into the brain is parallel to the orientation of the transmitters/receivers **26, 28.**

In the exemplary embodiment, the sensor includes a plurality of modules. Even if the transmitter/receiver **26, 28** of one module is not adequately oriented to measure the blood flow, the remaining transmitters/receivers **26, 28** may adequately cover the carotid artery or other selected vessel and be positioned to measure the flow of the blood cells. Furthermore, multiple modules **24** reduce the risk of improperly securing the ultrasound assembly **10** on the patient. Even if the ultrasound assembly **10** is positioned slightly off, the multiplicity of transmitters/receivers **26, 28** ensures that at least one pair is sufficiently positioned over the carotid artery to provide the means to detect the pulse.

In the manner described above, the hands free collection of ultrasound sensor data from a subject is performed. The above is applicable to a variety of ultrasound imaging modalities such as B-mode, Color Doppler, CW Doppler, M-Mode, imaging applications, and non imaging applications such as pulse detection during a CPR guided intervention during a cardiac resuscitation attempt. As an example, the described above ultrasound assembly can measure the actual bloodflow during CPR to provide feedback regarding the quality of the CPR.

For example, the CPR unit **52** transmits the digital signals to the ultrasound sensor **12** which trigger the transmitters **26** to emit CW signals to the blood cells in the carotid artery. The reflected signals are received by the receivers **28.** The return signals are indicative of the patient's pulse and thus, indicative of perfusion and are processed by the CPR unit to measure blood flow. For example, the return signals are compared with a threshold statistically appropriate with the returned signals received. If the return signals are above the threshold, then the return signals are indicative of a pulse and a rhythm is determined. Conversely, if the return signals are below the threshold, then the return signals are not considered a pulse but are considered to be background noise or low velocity residual flow. Based on the determined rhythm and absence of pulse, the CPR unit determines, for example, if performing CPR is advisable. The return signals may also be analyzed using appropriate signal processing methods including spectral analysis, correlation analysis and the like, in order to better infer the presence or absence of blood flow.

As another example, in nuclear medicine gating studies, such as cardiac imaging, the ultrasound assembly **10** is used to gate the data acquisition. The hands free ultrasound monitoring affords avoiding unnecessary exposure to radiation of the medical professional.

The apparatuses and methods described above are similarly applicable to animal imaging, monitoring, testing and treatment. Of course, it is contemplated that such apparatuses can be adopted for different size and/or anatomy of the animals, and the like.

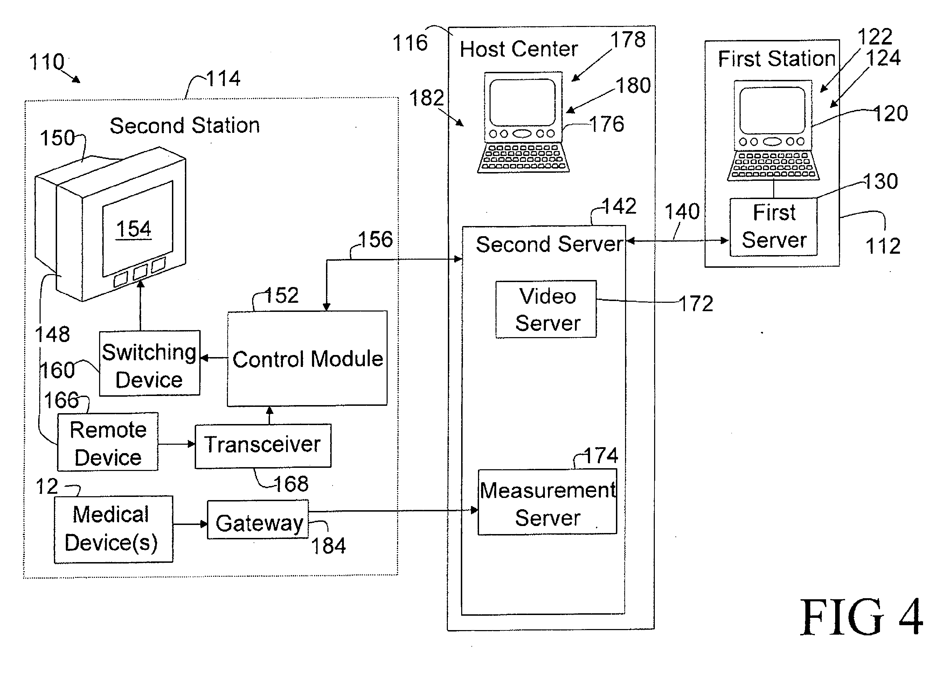

With reference again to FIGURE 1 and further reference to FIGURE 4, the ultrasound assembly **10** is used in a health management system **110** which includes first and second stations **112, 114** and a host center **116.** The first or care provider station **112** is located, for example, at a care provider site such as a physician's office or hospital and includes a terminal **120.** One example of the terminal **120** is a personal computer which includes appropriate software **122,** such as user interface software, and hardware **124,** for interfacing with the host center **116** and via the host center **116** with the second station **114.** The terminal **120** is connected to a first server **130** via an intranet or other connection as known in the art.

Of course, it is contemplated that the health management system **110** can include a plurality of the first stations **112,** a plurality of host centers **116** and a plurality of second stations **114** as appropriate for an application.

A first link **140** provides the connection between the first station **112** and the host center **116.** Alternatively, the first station **112** is a wireless station of a wireless local area network (LAN) or wireless wide are network (WAN).

The second or patient station **114** includes a user or patient interface **148** including a television set **150** or other patient display device which is located in a patient's home or dwelling. The user interface **148** further includes a control module, processor, algorithm or other means **152,** such as set-top box, which interfaces with a video display **154** of the television set **150.** The control module **152** converts and displays data from analog cable, digital cable, satellite, digital subscriber line (DSL), or digital broadcast television to a standard channel frequency, e.g. channel number, for display, for example, on a standard analog television set **150.** In another embodiment, the module **152** converts and displays the data directly on the television set **150** via an RCA (Radio Corporation of America) or SCART (Syndicat des Constructeurs d'Appareils Radiorécepteurs et Téléviseurs) connector or interface, without the need to encode the signal onto a television channel frequency. In one embodiment, the control module **152** further receives on or off-air digital or conventional analog television signals from a cable or satellite provider or local broadcast TV for display on a DTV monitor. The control module **152** also receives signals such as digital or analog television format signals and patient information signals from the host center **116** via a second link **156.** The examples of the second link **156** are wired connection, wireless connection, satellite connection, fiber optic connection, and the like.

The control module **152** is connected to the video display **154** via a switching device, algorithm or means **160** such as an audio/video (AV) switching device as known in the art. The switching device **160** provides switching between television reception from the tuner of the display **154** (or VCR, DVD or the like) and patient information reception/transmission from/to the host center **116.** Alternatively, any other known type of input device adapted to provide an interface to the video display **154** is used.

For example, the patient information signals include information, instructions and queries that are displayed on the video display for information, action, and the like. The patient information signals include video and audio health issue programs, audio programs, video messages and audio messages, reminders to send health or biometric information, and the like. The user interface **148** further includes a remote interface device **166** which provides signals to an infrared transceiver **168.** Signals from the transceiver **168** are provided to the control module **152** and function to select video input to the video display **154,** input patient information, and the like. In one embodiment, the remote interface device **166** is a remote control device such as one commonly used in the home entertainment systems. In another embodiment, the remote interface device **166** is a computer input interface device, such as a keyboard or a mouse.

The host center **116** is centralized and includes various servers for specific functions. The examples of servers of the host center **116** are a video server **172,** which provides pertinent video content to the display **154,** and a measurement server **174.** which collects and transfers patient's biometric measurements. The host center **116** includes a host center terminal **176** including appropriate hardware **178,** software **180** and communications links **182** to enable connectivity between the first and second stations **112, 114.**

It is also contemplated that the host center **116** is distributed, with different components or sub-centers hosting different functions. Alternatively, there may be a plurality of host centers **116** that connect a plurality of second stations **114** with one or more first stations **112.**

In one embodiment, the second station **114** includes a set of medical devices **12** associated with the patient **14.** E.g., in addition to the ultrasound assembly **10,** the medical devices **12** can include a weight scale, a blood pressure device, an electrocardiogram, an electroencephalogram, an oximeter, a brain wave measuring device, a respiration monitor, a thermometer, and the like. In one embodiment, the biometric devices **12** are wireless devices which are worn by the patient **14** and communicate biometric reading continuously or at intervals to the host center, or are cabled devices which the patient **14** uses one or more times a day to take readings, or the like. Additionally, or alternatively, certain measurements may be manually entered by the patient via the remote device **166.** Alternatively, the biometric device **12** can be implanted in the patient, such as a sensor on a pacemaker, on an infusion pump, and the like. Collected monitored or manual patient data are provided to a measurement gateway **184,** which transmits the data to the measurement server **174** for processing and use.

Other exemplary user interface devices are a personal computer (PC), personal digital assistant (PDA), a mobile phone, a portable computer, automated voice response system and the like. As such, the display is accordingly a computer monitor, handheld communication device display, such as a portable phone, cellular phone or PDA.

The following is also applicable in the defibrillator, after shock detect presence/absence of PEA to determine if CPR should be performed, monitoring in ambulance from emergency location to hospital, noninvasive cardiovascular monitoring in hospital bed, hands-free bloodflow monitoring during surgery, possibly in presence of radiation from other imaging modalities or therapeutic treatments.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

## Claims

1. A skin-mountable device **(10)** comprising:
an acoustic module **(24);** an attachment assembly **(16)** including an acoustically coupling layer **(34),** which affixes and acoustically couples the module **(24)** to a skin **(18);**
a flexible covering layer **(32)** which surroundingly extends over the module **(24)** to permit flexing for conforming to the contour of the skin;
an adhesive pad **(20, 22)** which extends peripherally around the coupling layer **(34);**
wherein the covering layer **(32)** covers an opposite side of the module **(24)** from the coupling layer **(34)** and an inner portion of the adhesive pad **(20, 22)** being sandwiched between the covering and coupling layers **(32, 34).**

2. The device as set forth in claim 1, further including a plurality of modules (**24**) to be affixed and acoustically coupled to the skin **(18)** by the coupling layer **(34).**

3. The device as set forth in claim 1, wherein the coupling layer **(34)** includes:
a dielectric silicone gel which is sufficiently tacky to adhere to the skin and sufficiently fluid to conform to the skin and fissures therein without air pockets.

4. The device as set forth in claim 1, wherein the acoustic module includes:
an acoustic transmitter element **(26),** an acoustic receiver element **(28),** and a former **(30)** which holds the acoustic transmitter and receiver elements in a fixed relationship to each other.

5. The device as set forth in claim 4, further including:
a plurality of the modules **(24)** flexibly interconnected between the acoustic coupling layer **(34)** on one side and said flexible covering layer **(32)** on the other side.

6. The device as set forth in claim 5, wherein the
pad **(20)** is integrated with which the covering and coupling layers **(32, 34).**

7. The device as set forth in claim 5, wherein the coupling layer (34) has a sufficiently low sound speed to cause beam refraction allowing the skin-mounted device **(10)** to be substantially flat.

8. The device as set forth in claim 1, wherein the module **(24)** includes an array of piezoelectric modules **(24)** each including:
a transmitter **(26)** which, in use, generates an ultrasonic transmission directed at a moving substance below the skin, and
a receiver **(28)** which, in use, senses reflected ultrasonic transmission which is indicative of the motion.

9. The device as set forth in claim 1, wherein the module **(24)** includes an array of piezoelectric modules **(24)** each including:
a transceiver **(26, 28)** which, in use, generates an ultrasonic transmission directed at a moving substance below the skin and senses reflected ultrasonic transmission which is indicative of the motion.

10. The device as set forth in claim 1, wherein the coupling layer **(34)** is a) sufficiently tacky to adhere the module **(24)** to the skin and hold the module in place without further human intervention during a medical procedure, b) sufficiently firm to prevent slippage, and c) sufficiently fluid to fill fissures in the skin as it is affixed.

11. A nuclear imager comprising the device of claim 1 to gate the data acquisition.

12. A method of acoustic monitoring with a device according to one of the claims 1 to 10 comprising:
affixing and acoustically coupling an acoustic module **(24)** with a patient's skin **(18)** with an acoustic coupling layer **(34);**
transmitting and receiving acoustic signals with the module to monitor a physiological parameter of the patient.

13. The method as set forth in claim 12, wherein the coupling layer **(34)** is a) sufficiently tacky to adhere the module **(24)** to the skin and hold the module in place without further human intervention during a medical procedure, b) sufficiently firm to prevent slippage, and c) sufficiently fluid to fill fissures in the skin as it is affixed.

14. An apparatus comprising:
a device according to one of the claims 1 to 10,
adapted for transmitting and receiving acoustic signals to monitor a physiological parameter of the patient.

15. A monitoring system **(110)** comprising:
a device according to one of the claims 1 to 10 which senses a physiological parameter of a patient **(14);** and
a server **(142)** which collects the sensed patient data and transfers the sensed patient data to a monitoring station **(52, 112, 116).**

## Patentansprüche

1. Auf der Haut zu befestigende Vorrichtung (10), die Folgendes umfasst:
ein akustisches Modul (24), eine Befestigungsanordnung (16) mit einer akustischen Kopplungsschicht (34), die das Modul (24) an Haut (18) befestigt und akustisch mit ihr koppelt,
eine flexible Deckschicht (32), die das Modul (24) umschließt, damit eine biegsame Anpassung an die Kontur der Haut ermöglicht wird,
ein Haftkissen (20, 22), das sich umfänglich um die Kopplungsschicht (34) erstreckt,
wobei die Deckschicht (32) eine der Kopplungsschicht (34) gegenüberliegende Seite des Moduls (24) bedeckt, und wobei ein innerer Teil des Haftkissens (20, 22) zwischen der Deckschicht und der Kopplungsschicht (32, 34) eingebettet ist.

2. Vorrichtung nach Anspruch 1, die ferner eine Vielzahl von Modulen (24) umfasst, die durch die Kopplungsschicht (34) an der Haut (18) zu befestigen und akustisch mit ihr zu koppeln sind.

3. Vorrichtung nach Anspruch 1, wobei die Kopplungsschicht (34) Folgendes umfasst:
ein dielektrisches Silikongel, das ausreichend klebrig ist, um an der Haut zu haften, und ausreichend flüssig ist, um sich der Haut und darin enthaltenen Rissen ohne Lufteinschlüsse anzupassen.

4. Vorrichtung nach Anspruch 1, wobei das akustische Modul Folgendes umfasst:
einen akustischen Sender (26), einen akustischen Empfänger (28) und einen Träger (30), der den akustischen Sender und den akustischen Empfänger in einer ortsfesten Beziehung zueinander hält.

5. Vorrichtung nach Anspruch 4, die ferner Folgendes umfasst:
eine Vielzahl von Modulen (24), die zwischen der akustischen Kopplungsschicht (34) auf der einen Seite und der genannten flexiblen Deckschicht (32) auf der anderen Seite flexibel miteinander verbunden sind.

6. Vorrichtung nach Anspruch 5, wobei das Kissen (20) in die Deckschicht und die Kopplungsschicht (32, 34) integriert ist.

7. Vorrichtung nach Anspruch 5, wobei die Kopplungsschicht (34) eine ausreichend niedrige Schallgeschwindigkeit hat, um eine Strahlbrechung zu verursachen, so dass die auf der Haut zu befestigende Vorrichtung (10) im Wesentlichen flach sein kann.

8. Vorrichtung nach Anspruch 1, wobei das Modul (24) eine Matrix aus piezoelektrischen Modulen (24) umfasst, die jeweils Folgendes umfassen:
einen Sender (26), der beim Einsatz Ultraschallwellen erzeugt, die auf eine sich unter der Haut bewegende Substanz gerichtet sind, und
einen Empfänger (28), der beim Einsatz reflektierte Ultraschallwellen misst, die auf die Bewegung hindeuten.

9. Vorrichtung nach Anspruch 1, wobei das Modul (24) eine Matrix aus piezoelektrischen Modulen (24) umfasst, die jeweils Folgendes umfassen:
einen Transceiver (26, 28), der beim Einsatz Ultraschallwellen ?? erzeugt, die auf eine sich unter der Haut bewegende Substanz gerichtet sind, und reflektierte Ultraschallwellen misst, die auf die Bewegung hindeuten.

10. Vorrichtung nach Anspruch 1, wobei die Kopplungsschicht (34) a) ausreichend klebrig ist, um das Modul (24) an der Haut haften zu lassen und das Modul während eines medizinischen Verfahrens ohne weiteres menschliches Eingreifen an Ort und Stelle zu halten, b) ausreichend fest ist, um ein Verrutschen zu verhindern, und c) ausreichend flüssig ist, um beim Befestigen Risse in der Haut auszufüllen.

11. Nukleare Bildgebungseinrichtung mit der Vorrichtung aus Anspruch 1, um die Datenerfassung einem Gating zu unterziehen.

12. Verfahren der akustischen Überwachung mit einer Vorrichtung nach einem der Ansprüche 1 bis 10, das Folgendes umfasst:
Befestigen und akustisches Koppeln eines akustischen Moduls (24) mit der Haut (18) eines Patienten über eine akustische Kopplungsschicht (34),
Senden und Empfangen von akustischen Signalen mit dem Modul, um einen physiologischen Parameter des Patienten zu überwachen.

13. Verfahren nach Anspruch 12, wobei die Kopplungsschicht (34) a) ausreichend klebrig ist, um das Modul (24) an der Haut haften zu lassen und das Modul während eines medizinischen Verfahrens ohne weiteres menschliches Eingreifen an Ort und Stelle zu halten, b) ausreichend fest ist, um ein Verrutschen zu verhindern, und c) ausreichend flüssig ist, um Beim Befestigen Risse in der Haut auszufüllen.

14. Gerät, das Folgendes umfasst:
eine Vorrichtung nach einem der Ansprüche 1 bis 10, die so ausgelegt ist, dass sie zum Überwachen eines physio logischen Parameters des Patienten akustische Signale sendet und empfängt.

15. Überwachungssystem (110), das Folgendes umfasst:
eine Vorrichtung nach einem der Ansprüche 1 bis 10, die einen physiologischen Parameter eines Patienten (14) misst, und
einen Server (142), der die gemessenen Patientendaten sammelt und die gemessenen Patientendaten zu einer Überwachungsstation (52, 112, 116) weiterleitet.

## Revendications

1. Dispositif qui peut être installé sur la peau (10) comprenant :
un module acoustique (24) ; un ensemble de fixation (16) comprenant une couche de couplage acoustique (34) qui fixe et couple acoustiquement le module (24) à une peau (18) ;
une couche de revêtement flexible (32) qui s'étend de manière environnante sur le module (24) pour permettre de fléchir pour s'adapter au contour de la peau ;
une compresse adhésive (20, 22) qui s'étend de manière périphérique autour de la couche de couplage (34) ;
dans lequel la couche de revêtement (32) recouvre un côté opposé du module (24) depuis la couche de couplage (34) et une partie interne de la compresse adhésive (20, 22) qui est intercalée entre les couches de revêtement et de couplage (32, 34).

2. Dispositif selon la revendication 1, comprenant en outre une pluralité de modules (24) qui doivent être fixés et couplés de manière acoustique à la peau (18) par la couche de couplage (34).

3. Dispositif selon la revendication 1, dans lequel la couche de couplage (34) comprend :
un gel de silicone diélectrique qui est suffisamment collant pour adhérer à la peau et suffisamment fluide pour s'adapter à la peau et aux fissures dans celle-ci sans poches d'air.

4. Dispositif selon la revendication 1, dans lequel le module acoustique comprend :
un élément émetteur acoustique (26), un élément récepteur acoustique (28) et un gabarit (30) qui maintient les éléments émetteur et récepteur acoustiques dans une relation fixe l'un par rapport à l'autre.

5. Dispositif selon la revendication 4, comprenant en outre :
une pluralité de modules (24) interconnectés de manière flexible entre la couche de couplage acoustique (34) sur un côté et ladite couche de revêtement flexible (32) sur l'autre côté.

6. Dispositif selon la revendication 5, dans lequel la compresse (20) est intégrée dans les couches de revêtement et de couplage (32, 34).

7. Dispositif selon la revendication 5, dans lequel la couche de couplage (34) a une vitesse sonore relativement faible pour provoquer une réfraction de faisceau permettant au dispositif installé sur la peau (10) d'être sensiblement plat.

8. Dispositif selon la revendication 1, dans lequel le module (24) comprend un ensemble de modules piézoélectriques (24), chacun comprenant :
un émetteur (26) qui, lors de l'utilisation, génère une transmission ultrasonore dirigée vers une substance mobile sous la peau, et
un récepteur (28) qui, lors de l'utilisation, détecte une transmission ultrasonore réfléchie qui est indicative du mouvement.

9. Dispositif selon la revendication 1, dans lequel le module (24) comprend un ensemble de modules piézoélectriques (24), chacun comprenant :
un émetteur-récepteur (26, 28) qui, lors de l'utilisation, génère une transmission ultrasonore dirigée vers une substance mobile sous la peau et détecte une transmission ultrasonore réfléchie qui est indicative du mouvement.

10. Dispositif selon la revendication 1, dans lequel la couche de couplage (34) est a) suffisamment collante pour faire adhérer le module (24) à la peau et maintenir le module en place sans intervention humaine supplémentaire pendant un acte médical, b) suffisamment ferme pour empêcher un glissement et c) suffisamment fluide pour boucher les fissures dans la peau à mesure qu'elle est fixée.

11. Dispositif d'imagerie nucléaire comprenant le dispositif de la revendication 1 pour déclencher l'acquisition de données.

12. Procédé de surveillance acoustique avec un dispositif selon l'une quelconque des revendications 1 à 10, comprenant les étapes consistant à :
fixer et coupler de manière acoustique un module acoustique (24) à la peau (18) d'un patient avec une couche de couplage acoustique (34) ;
transmettre et recevoir des signaux acoustiques avec le module pour surveiller un paramètre physiologique du patient.

13. Procédé selon la revendication 12, dans lequel la couche de couplage (34) est a) suffisamment collante pour faire adhérer le module (24) à la peau et maintenir le module en place sans intervention humaine supplémentaire pendant un acte médical, b) suffisamment ferme pour empêcher un glissement et c) suffisamment fluide pour boucher les fissures dans la peau à mesure qu'elle est fixée.

14. Appareil comprenant :
un dispositif selon l'une quelconque des revendications 1 à 10,
conçu pour transmettre et recevoir des signaux acoustiques pour surveiller un paramètre physiologique du patient.

15. Système de surveillance (110) comprenant :
un dispositif selon l'une quelconque des revendications 1 à 10 qui détecte un paramètre physiologique d'un patient (14) ; et
un serveur (142) qui recueille les données de patient détectées et transfère les données de patient détectées à un poste de surveillance (52, 112, 116).
